# EUROPEAN PATENT APPLICATION

(11) **EP 3 091 031 A1**
(43) Date of publication of application: **09.11.2016**
(21) Application number: 15166303.6
(22) Date of filing: 04.05.2015
(51) Int. Cl.: C07K 16/28, A61P 35/00, A61K 39/00

(54) **COMBINATION OF A BISPECIFIC ANTIBODY WITH AN IMMUNE MODULATING MOLECULE FOR THE TREATMENT OF A TUMOR**

(71) Applicant: Affimed GmbH, 69120 Heidelberg (DE)
(72) Inventor: Treder, Martin, Dr., 69120 Heidelberg (DE); Reusch, Uwe, Dr., 67487 Maikammer (DE); Marschner, Jens-Peter,, 64342 Seeheim-Jugenheim (DE); Knackmuss, Stefan, Dr., 68723 Plankstadt (DE); Holbrook, Kohrt,, Stanford, CA 94305-5151 (US)
(74) Representative: Schüssler, Andrea

(57) **Abstract**

Described is a combination of (i) a multifunctional antibody having specificity for an antigen present on a tumor cell and having specificity for an antigen present on a natural killer (NK) cell and (ii) at least one agent modulating an immune checkpoint molecule for the treatment of a tumor. The antibody may be a bispecific CD30/CD16 antibody and the agent can be anti-CTLA-4, anti-PD1 and ant-CD 137.

## Description

The invention relates to a combination of (i) a multifunctional antibody having specificity for an antigen present on a tumor cell and having specificity for an antigen present on a natural killer (NK) cell and (ii) at least one agent being an immunomodulatory molecule for the treatment of a tumor.

This combination results in an enhanced tumor cell killing, because the combination of an NK cell engaging and tumor targeting multifunctional antibody with the immune modulating agent has a synergistic anti-tumor effect through an orchestrated immune response involving both NK-cells and T-cells. In preclinical animal studies of Hodgkin lymphoma using both patient derived xenograft (PDX) and immune cells from blood (PBMC), the established tumor was treated with a bispecific CD30/CD16 antibody in combination with an agent modulating an immune checkpoint molecule (anti-PD1, anti-CD137 and anti-CTLA4) both alone and in combination. While the single agent treatment showed a significant reduction in tumor growth for most molecules when compared to the control treatment group (irrelevant IgG), all combinations of bispecific CD30/CD16 multifunctional antibody and agents modulating an immune checkpoint molecule showed enhanced anti-tumor efficacy. Compared to IgG treatment it was observed that in animals treated with combinations of CD30/CD16 and an agent modulating an immune checkpoint molecule the NK cell population in the tumor increased. In addition, while there was no increase of T-cells in animals treated only with CD30/CD16 or an agent modulating an immune checkpoint molecule alone, the cytotoxic T-cells detected in animals treated with CD30/CD16 in combination with an agent modulating an immune checkpoint molecule increased.

Therefore, the combination of (i) a multifunctional antibody having specificity for an antigen present on a tumor cell and having specificity for an antigen present on a natural killer (NK) cell and (ii) at least one agent being an immunomodulatory molecule, e.g. checkpoint inhibitor or agonist, increases synergistically the killing of tumor cells which results in a significantly increased tumor regression. The remarkable tumor regression is achieved by the coordinated action of T- and NK-cells through the combined activity of the NK-cell engaging multifunctional antibody and the agent modulating the immune checkpoint molecule. Immune checkpoint molecules are cell surface proteins, e.g. receptors that regulate costimulatory or coinhibitory pathways of the immune response.

Examples of immune checkpoint molecules are cytotoxic T-lymphocyte antigen 4 (CTLA-4), programmed death-1 (PD-1), programmed death ligand-1 (PD-L1), immune costimulatory molecules on NK cell and costimulatory receptors of the TNF receptor family, for example CD137.

The agent being an immunomodulatory molecule can be an agent for modulating an immune checkpoint molecule, for example an antagonistic antibody blocking a coinhibitory pathway or an agonistic antibody inducing a costimulatory pathway through binding to the respective immune checkpoint molecule. Such antibodies are also known as checkpoint inhibitors (CPI) or checkpoint agonists (CPA) and have been described and clinically tested.

Examples of immune checkpoint molecules are CTLA-4, PD-1, PD-L1, and CD137.

CTLA-4 induces a signal that inhibits T-cell response. Examples of CTLA-4 antibodies are ipilimumab and tremelimumab.

PD-1 (PDCD1 or CD279) receptor mediates a coinhibitory pathway. Further PD-1 binds to PD-L1 which induces a coinhibitory signaling upon receptor-ligand ligation. Examples of PD-1 antibodies include nivolumab, MK3475 and pidilizumab.

CD137 (4-1BB) or TNF receptor superfamily 9 (TNFRSF9) is a costimulatory receptor that belongs to the TNF receptor superfamily, a member of tumor necrosis factor receptor superfamily which is involved in the regulation of the activation of immune cells. The functional role of CD137 is enhancing cytotoxic T cell responses. An example of a CD137 agonistic antibody which enhances the T cell response is urelumab.

A multifunctional antibody having specificity for an antigen present on a tumor cell and having specificity for an antigen present on a natural killer (NK) cell can be used for NK-cell based immunotherapy of a tumor.

The term multifunctional as used herein means that an antibody exhibits two or more different biological functions. For example, the different biological functions are different specificities for different antigens. In certain instances, the multifunctional antibody is bispecific. Such bispecific binding proteins include, for example, bispecific monoclonal antibodies of the classes IgA, IgD, IgE, IgG or IgM, as well as antibody fragments or antibody derivatives including, for example, Fab, Fab', F(ab')₂, Fv fragments, single-chain Fv, tandem single-chain Fv (scFv)₂, Bi-specific T-cell engagers (BiTE®), dual affinity retargeting antibodies (DART™), diabody and tandem diabody (TandAb®), single-chain diabodies (scDb) and flexibodies. The term antibody as used herein means monoclonal antibodies as well as antibody fragments and antibody derivatives comprising an antibody binding domain. Examples of bispecific antibodies are described in Spiess, C. et al., Alternative molecular formats for bispecific antibodies. Mol Immunol (2015), http://dx.doi.org/10.1016/j.molimm.2015.01.003 and Kontermann, R.E., Brinkmann, U. Bispecific antibodies, Drug Discov. Today (2015), http://dx.doi.org/10.1016/j.drugdis.2015.02.008

In certain embodiments the multifunctional antibody is a bispecific tandem diabody (TandAb®). A tandem diabody is constructed by linking the four variable domains of the heavy and light chains (VH and VL) from two different Fv in a single polypeptide. The domains are positioned such that corresponding VH and VL can pair when two molecules of the polypeptide align in a head-to-tail fashion. Short linkers between the domains (twelve or fewer amino acids) prevent intramolecular pairing of the FV. The tandem diabody and its manufacture is described in Weichel et al., TandAbs: potent and well-manufacturable bi-specific antibodies; European Pharmaceutical Review 2015, vol. 20:27-32, Kipriyanov SM: Methods Mol. Biol. 2009;562:177-93 or Kipriyanov SM: Methods Mol Biol 2003;207:323-33.

In certain embodiments first the multifunctional antibody is administered and subsequently the agent being an immunomodulatory molecule is administered. Administration of the multifunctional antibody and the agent being an immunomodulatory molecule can be effected by different ways, e.g. by intravenous, intraperitoneal, subcutaneous, intramuscular, topical or intradermal administration. In some embodiments, the route of administration depends on the kind of therapy and the kind of compound contained in the pharmaceutical composition. The dosage regimen will be determined by the attending physician and other clinical factors. Dosages for any one patient depends on many factors, including the patient's size, body surface area, age, sex, the particular compound to be administered, time and route of administration, the kind of therapy, general health and other drugs being administered concurrently. An "effective dose" refers to amounts of the active ingredient that are sufficient to affect the course and the severity of the disease, leading to the reduction or remission of such pathology. An "effective dose" useful for treating a tumor may be determined using known methods.

This immunotherapeutic approach of antibody-mediated recruitment of NK cells to tumors using multifunctional antibodies can be used for the treatment of hematological or solid tumors.

In certain embodiments such multifunctional antibody is a dual-targeting bispecific antibody which recruits NK cells via their activating receptor to tumor cells. One antibody specificity is anti-CD16, whereas the other specificity is selected to target an antigen present on a tumor cell, e.g. a tumor specific antigen.

In certain embodiments the multifunctional antibody recruits NK cells by binding exclusively to the CD16 isoform CD16A. Examples of anti-CD16A binding domains are described in WO 2006/125668.

The specificity of the multifunctional antibody for an antigen present on a tumor cell may be a tumor antigen or a cell surface antigen on the respective tumor cell, for example a specific tumor marker. The term tumor antigen present on a tumor cell as used herein comprises tumor associated antigen (TAA) and tumor specific antigen (TSA).

Examples for an antigen present on a tumor cell but not limited thereto according to the invention are CD19, CD30, EGFR or EGFRvIII. Thus, examples of a multifunctional antibody according to the invention are the bispecific antibodies anti-CD 19/anti-CD16A, anti-CD30/anti-CD16A, anti-EGFR/anti-CD16A and anti-EGFRvIII/anti-CD16A.

In an embodiment a combination of a multifunctional, e.g. bispecific, CD30/CD16 antibody, e.g. CD30/CD16A, and at least one agent being an immunomodulatory molecule is used for treating CD30+ tumors, such as, for example, Hodgkin lymphoma or anaplastic large-cell lymphoma (ALCL).

In certain embodiments first the CD30/CD16 antibody is administered and subsequently the agent being an immunomodulatory molecule is administered. For example, the agent being an immunomodulatory molecule 10-72 h, e.g. 1 day, after the administration of the CD30/CD16 antibody.

Examples of agents modulating an immune checkpoint molecule used in combination with the CD30/CD16 antibody are the antibodies anti-CTLA-4, anti-PD-1 and anti-CD 137. Preferred are anti-PD-1 and anti-CD 137.

In a particular embodiment, both antibodies anti-PD-1 and anti-CD137 are administered in combination with the bispecific CD30/CD16 antibody.

In a certain embodiment of the invention the multifunctional antibody is a bispecific tetravalent tandem diabody CD30/CD16A. Example 1 describes a CD30/CD16A tandem diabody which specifically recruits NK cells by binding exclusively to the isoform CD16A. Tandem diabodies have two binding sites for each antigen, but no Fc domains. The CD30/CD16A tandem diabody of example 1 has a molecular weight of 104 kDa and can be produced in bacteria or in mammalian cells, e.g. CHO. It specifically targets CD30 on Hodgkin lymphoma cells and recruits and activates NK-cells by binding to CD16A. The efficacy of this CD30/CD16A tandem diabody is reported in Rothe et al., A phase I study of the bispecific CD30/CD16A antibody construct AFM13 in patients with relapsed or refractory Hodgkin lymphoma; Blood. 2015 Apr 17. pii: blood-2014-12-614636.

### Brief description of the drawings:

- Figure 1: shows a chromium release assay. Percent lysis was determined after cultures of pre-activated, purified NK cells at variable effector:target cell (Karpas-299) ratios with ⁵¹Cr-labeled lymphoma cells in media alone (no antibody), or with single (aCD137, aPD1, aCTLA4) or multiple antibodies (aCD137 and aPD1).
- Figure 2: shows a chromium release assay. Percent lysis was determined after cultures of pre-activated, purified NK cells at variable effector:target cell (Karpas-299) ratios with ⁵¹Cr-labeled lymphoma cells in media alone (no antibody), or with single (aCD137, aPD1, aCTLA4) or multiple antibodies (aCD137 and aPD1), CD30/CD16A tandem diabody was used at 1 pM concentrations.
- Figure 3: shows results of an *in vivo* PDX model. AFM13 designates CD30/CD16A tandem diabody
- Figure 4: shows results of an *in vivo* PDX model. AFM13 designates the CD30/CD16A tandem diabody.
- Figure 5: *In vivo* PDX model: increased lymphocytes in CD30/CD16A tandem diabody treated mice

### Example 1:

### CD137 co-stimulation and blocking PD-1 enhances NK cell-mediated target cell lysis by bispecific CD30/CD16A tandem diabody

### Methods:

Efficacy was assessed by in vitro cytotoxicity with human PBMCs, enriched NKs, and CD30⁺ target cells as well as cell line and patient-derived xenograft in vivo models with CD30/CD16A tandem diabody, anti-CTLA-4, anti-PD-1, or anti-CD 137 antibodies.

To evaluate NK cell cytotoxicity for CD30⁺ lymphoma cell lines, chromium release was performed as follows: PBMCszz were cultured for 24 hours together with anti-CD30 (10 µg/mL) and irradiated (5,000 rads) CD30⁺ lymphoma tumor cells_at a ratio of 1:1. After 24 hours, NK cells were isolated from these cultures by negative magnetic cell sorting using NK cell isolation beads (Miltenyi Biotec) according to manufacturer's instructions. NK cells were assessed for purity (>90% purity as defined by flow cytometry) prior to chromium release assay. Target cells were labeled with 150 µCi ⁵¹Cr per 1x10⁶ cells for 2 hours. Percent lysis was determined after 4 hour cultures of pre-activated, purified NK cells at variable effector:target cell ratios with ⁵¹Cr-labeled lymphoma cells in media alone, or with single or multiple antibodies.

Xenografted tumor pieces (8x8mm) derived from a surgical specimen of a newly diagnosed patient with CD30⁺ lymphoma (including Hodgkin Disease), in Rag2^{-/-}IL2Rγ^{null} mice (n∼100) were observed for engraftment and up to 80 mice with engraftment of similar size (0.5cm²) randomized into up to 8 groups on day 28. Autologous PBMCs were infused on day 28 (2x10⁶ PBMCs/mouse) intra-peritoneally. Therapy begins on day 28 and is continued weekly for a total of three intraperitoneal injections, all dosed at 15mg/kg. With combination therapy, anti-CD30/CD16a was dosed on day 28 and anti-CTLA4 (Ipilimumab), anti-CD137 (Urelumab) or anti-PD1 (Pembrolizumab) dosed on day 29. Tumor size was compared between groups at day 56. All mice are sacrificed for immunophenotyping once a group requires euthanasia due to growth to 700% of original tumor size (app 3.5cm²) on day 58.

The CD30/CD16A tandem diabody is the antibody AFM13 described in Rothe et al., A phase I study of the bispecific CD30/CD16A antibody construct AFM13 in patients with relapsed or refractory Hodgkin lymphoma; Blood. 2015 Apr 17. pii: blood-2014-12-614636. The CD30/CD16A tandem diabody comprises the anti-CD30 domain of hybridoma HRS-3 and its construction and expression in bacteria is disclosed in example 19 of WO 2006/125668.

### Results:

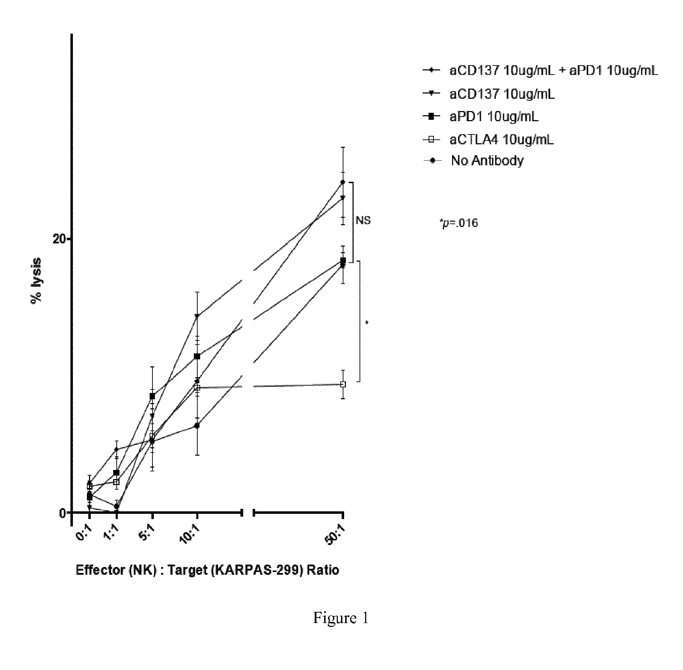

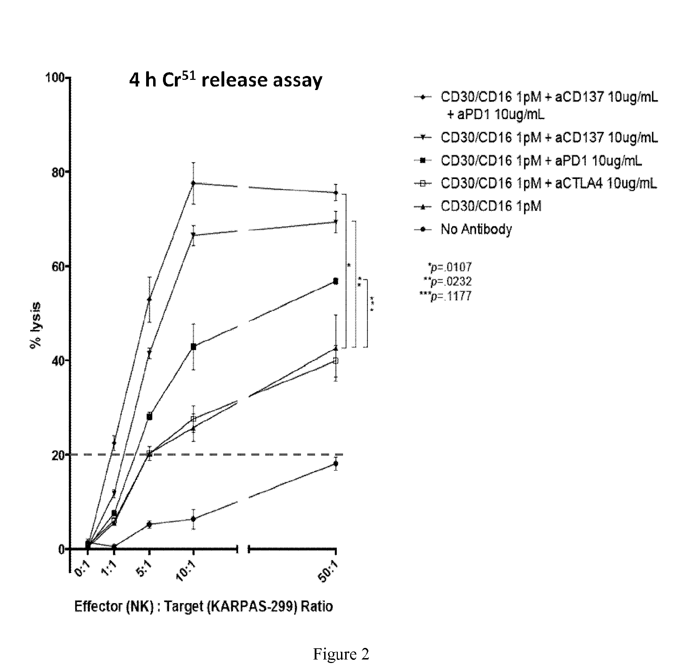

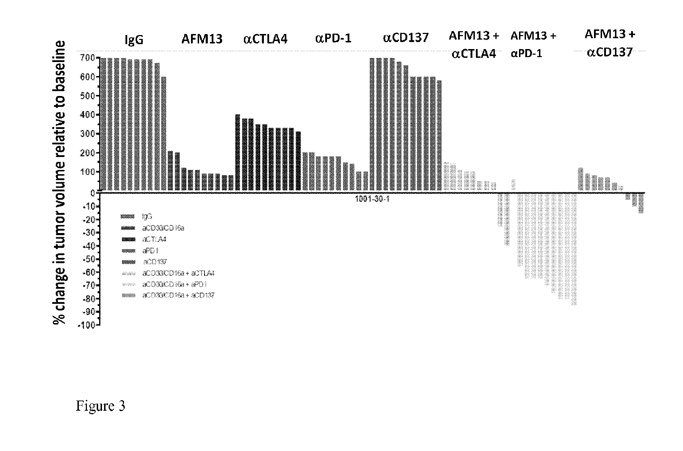

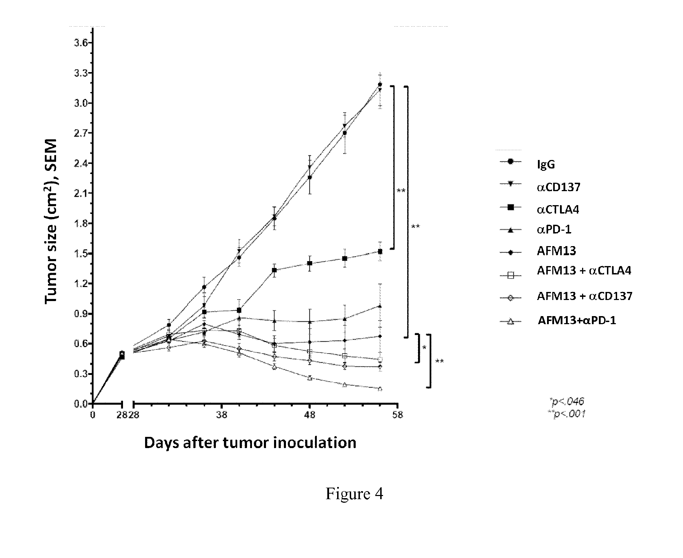

CD30/CD16A tandem diabody demonstrated higher potency and efficacy toward target and effector cells relative to other CD30⁺ antibody formats (EC50 = 15pM). These favorable properties resulted in superior cytotoxicity when CD30/CD16A tandem diabody was incubated with CD30⁺ tumor cells and enriched NK cells (Figure 2). Single treatment with CD30/CD16A tandem diabody at suboptimal concentrations (1 pM) induced effector-to-target cell-dependent lysis of CD30⁺ lymphoma cells up to 40% using enriched NK cells. Immune-modulating antibodies alone mediated substantially lower lysis (<25%) (Figure 1). However, the addition of anti-PD-1 or anti-CD137 to CD30/CD16A tandem diabody strongly enhanced specific lysis up to 70%, whereas the addition of anti-CTLA-4 to CD30/CD16A tandem diabody showed no beneficial effect. The most impressive increase of efficacy was observed when CD30/CD16A tandem diabody was applied together with a combination of anti-PD-1 and anti-CD137 (Figure 2). In vivo, synergy of CD30/CD16A tandem diabody and immune modulating antibody combination was observed with each immune modulating antibody tested and augmented with anti-PD1 (regression in 9/10 tumors), ant-CTLA-4 (3/10), and anti-CD137 mAb (3/10) and influenced by presence of regulatory T cells, NK cells, and Th1 cytokines (Figures 3 and 4).

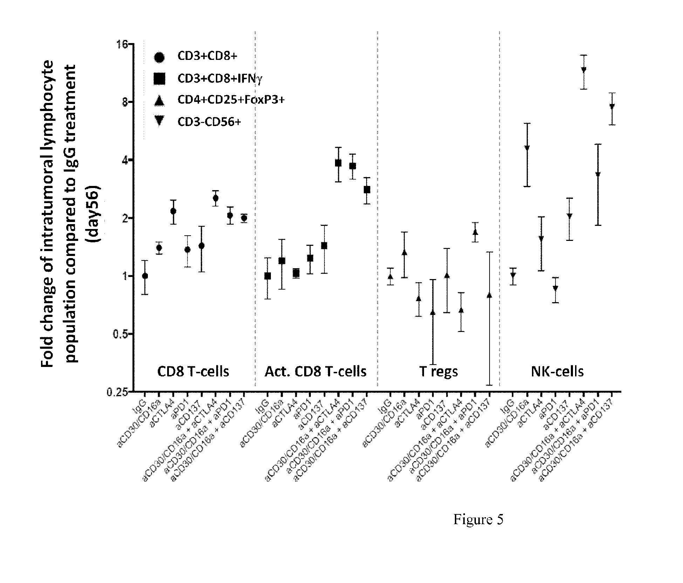

Compared to IgG treatment it was observed that in animals treated with combinations of CD30/CD16A tandem diabody and anti-CTLA-4, anti-PD-1 and anti-CD137 the NK cell population in the tumor increased. In addition, while there was no increase of T-cells in animals treated only with CD30/CD16A tandem diabody or anti-CTLA-4, anti-PD-1 and anti-CD137 alone, the cytotoxic T-cells detected in animals treated with CD30/CD16A tandem diabody in combination with anti-CTLA-4, anti-PD-1 and anti-CD137 increased (Figure 5).

The findings support that dual-antibody therapy augments the efficacy of CD30/CD16A tandem diabody and immune modulating antibodies achieving a remarkable tumor regression.

## Claims

1. A combination of a multifunctional antibody and at least one agent modulating an immune checkpoint molecule, wherein the multifunctional antibody has specificity for an antigen present on a tumor cell and has specificity for an antigen present on a natural killer (NK) cell for use in a method of treating a tumor.

2. The combination of claim 1, wherein the cancer is a solid tumor or a haematological tumor.

3. The combination of claim 2, wherein the haematological tumor is a Hodgkin lymphoma.

4. The combination of claim 3, wherein the multifunctional antibody has specificity for CD30.

5. The combination of any one of the claims 1-4, wherein the multifunctional antibody has specificity for CD16.

6. The combination of claim 5, wherein the multifunctional antibody has specificity for CD16A.

7. The combination of claim 6, wherein the multifunctional antibody is a bispecific antibody having specificity for CD16A and CD30.

8. The combination of any one of claims 1-6, wherein the immune checkpoint molecule is an immune costimulatory molecule on a NK cell.

9. The combination of any one of claims 1-7, wherein the agent modulating an immune checkpoint molecule is an antibody selected from the group consisting of anti-CD 137, anti-PD1 and anti-CTLA4.

10. The combination of any one of the claims 1-9, wherein two agents modulating immune checkpoint molecules are used.

11. The combination of claim 10, wherein anti-CD 137 and anti-PD1 are used.

12. The combination of any one of claims 1-11, wherein the multifunctional antibody is administered prior to the agent modulating an immune checkpoint molecule.

13. The combination of any one of claims 6-12, wherein the multifunctional antibody is a tetravalent bispecific CD30/CD16A tandem diabody.
